# EUROPEAN PATENT APPLICATION

(11) **EP 2 599 483 A1**
(43) Date of publication of application: **05.06.2013**
(21) Application number: 11009478.6
(22) Date of filing: 30.11.2011
(51) Int. Cl.: A61K 31/192, A61K 9/20, A61K 9/28, A61K 47/18

(54) **4-Phenylbutyric acid formulation**

(71) Applicant: Lunamed AG, 7000 Chur (CH)
(72) Inventor: The designation of the inventor has not yet been filed
(74) Representative: Peters, Hajo

(57) **Abstract**

The present invention refers to a pharmaceutical composition for 4-phenylbutyric acid or its ions and a compound comprising a L-aspartate-D-alanine core structure. The pharmaceutical composition include organic salts of phenylbutyrate, polymorphs, hydrates or solvates thereof and co-crystals of phenylbutyric acid and at least one organic molecule, polymorphs, hydrates or solvates thereof, as well as their use in cancer therapy and other pharmaceutical applications.

## Description

### Field of the invention

The present invention relates to a pharmaceutical composition comprising 4-phenylbutyric acid (4-PBA) or its ions and a compound comprising an L-Asp-D-Ala core structure which is preferably alitame. Said pharmaceutical composition includes a co-crystal comprising 4-phenylbutyric acid or its ions and a compound comprising an L-Asp-D-Ala core structure which is preferably alitame. The invention relates further to the therapeutic use of said pharmaceutical compositions.

### Background of the invention

Phenylbutyric acid is a well known compound to which a number of medical uses have been ascribed in patent or scientific literature. In form of its sodium salt it is marketed as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

Sodium 4-phenylbutyrate which is normally given orally is a very bitter compound and has a pungent odor of mice. This combination makes the compound very unacceptable to patients who have to take large amounts of the medicine every day. This tends to lead to lack of compliance in taking the prescribed dose at the required intervals.

The severity of this problem is also emphasized in a publication of George J. Dover (Blood, Vol. 84, No. 1, Jul. 1, 1994: pp 339-343) relating to the use of 4-PBA for treatment of sickle cell anemia where it is stated:
*"[Sodium phenylbutyrate] in powder form has a bitter taste that, despite many attempts, cannot be disguised. Two of the three subjects treated after discharge from the hospital reported inability to maintain compliance."*

This led to the development of 4-PBA formulation comprising synthetic sweetening agents.

US Pat. App. 2004/0152784 describe a pharmaceutical composition of sodium 4-phenylbutyrate with effective aromatic flavoring agent and at least one synthetic sweetening agent. This disclosure provides a dry granulated pharmaceutical composition that can be dissolved in water before administration. Noticeably, this application also demonstrates that the sweetening agent (potassium aspartame) is not stable in the aqueous reconstituted solution of the dry powder containing sodium 4-phenylbutyrate because it loses its sweetness when stored for more than a few weeks.

WO 2007/005633 discloses a liquid formulation of 4-PBA comprising saccharine as sweetening agent. However, saccharin has a bitter or metallic aftertaste, especially at high concentrations.

There is thus a need to provide an improved pharmaceutical composition containing 4-phenylbutyric acid or its salts for use in the treatment of patients which represents a pharmacologically acceptable form of sodium 4-phenylbutyrate and which improves patient compliance.

Accordingly, it was the goal of this invention to identify alternative pharmaceutical composition for phenylbutyric acid or its ions which overcome at least some of the disadvantages of the above disclosed formulations.

It has now been found that pharmaceutical composition comprising 4-phenylbutyric acid and a peptidic compound comprising a L-aspartate-D-alanine core structure represents a pharmacologically acceptable composition while efficiently masking the bitter taste of 4-PBA.

Accordingly, the invention is drawn to a pharmaceutical composition comprising as a component A represented by a substance comprising an L-aspartate-D-alanine dipeptide or a salt or a solvate thereof; and as a component B represented by 4-phenyl-butyric acid or a salt or solvate thereof.

Most dipeptides are not sweet, but the unexpected discovery of aspartame in 1965 led to a search for similar compounds that shared its sweetness. This led to the discovery of improved peptidic sweeteners comprising an L-aspartyl-D-alanine dipeptide core.

Peptidic Sweeteners comprising an L-aspartyl-D-alanine dipeptide core exhibit several advantages:
□ They represent biocompatible and safe sweeteners which can be given in high doses without health impairments.
□ Unlike aspartame they do not possess phenylalanine, which is contraindicated for patients suffering from phenylketonuria.
□ The metabolic degradation of such peptidic sweeteners leads to the generation of D-alanine which is sweet in itself.
□ D-alanine is not processed from the body and therefore does not increase the calorie content of the composition.
□ Since the sweetener contains the D-amino acid D-alanine it shows increased resistance to metabolic degradation which improves the stability of the respective sweetener.
□ Most of the L-Asp-D-alanine based peptidic compounds are potent sweeteners with a refined taste. For example alitame is about 2000 times sweeter than sucrose, about 10 times sweeter than aspartame, and has no aftertaste.

A "pharmaceutical composition" is defined as any composition, other than food, used in the prevention, diagnosis, alleviation, treatment, or cure of disease in man and animal. "Pharmaceutical compositions" include a) a salt of phenylbutyrate with a component A as a charged ion including a respective polymorph, hydrate or solvate of the salt; b) a salt of phenylbutyrate with a positively charged ion of component A; c) a polymorph of a salt of phenylbutyrate with a charged ion of compound A; d) a hydrate of a salt of phenylbutyrate with a charged ion of compound A; e) a solvate of a salt of phenylbutyrate with a charged ion of compound A; f) a co-crystal of phenylbutyric acid and compound A as co-crystal former, including a respective polymorph, hydrate or solvate of the co-crystal; g) a co-crystal of phenylbutyric acid and compound A as co-crystal former; h) a polymorph of a co-crystal of phenylbutyric acid and compound A as co-crystal former; i) a hydrate of a co-crystal of phenylbutyric acid and compound A as co-crystal former; or j) a solvate of a co-crystal of phenylbutyric acid and compound A as co-crystal former.

The term "salt" is to be understood as meaning any form of the compound A or the compound B used according to the invention in which it assumes an ionic form or is charged and is coupled with a counter-ion (a cation or anion) or is in solution. By this are also to be understood complexes of the compound A or compound B with other molecules and ions, in particular complexes which are complexed via ionic interactions.

Within the context of the invention a "solvate" is defined as any form of compound A or compound B in which this compound has attached to it via non-covalent binding another molecule (most likely a polar solvent) especially including hydrates and alcoholates, e.g. methanolate or ethanolate.

### Detailed description of the invention

In a first aspect the invention relates to pharmaceutical composition comprising as a component A: a substance comprising an L-aspartate-D-alanine dipeptide or a salt or a solvate thereof; and as a component B: 4-phenyl-butyric acid or a salt or solvate thereof.

Within compound A, the L-aspartate-D-alanine dipeptide can represent the N-terminal or C-terminal end of the respective substance; however, it can also represent an integral peptidic core with further groups, preferably amino acids, attached to the free amino- and carboxyl group of said dipeptide.

In a further embodiment of the invention said L-aspartate-D-alanine dipeptide is part of a larger peptide or protein.

A "protein" in the context of the invention is defined as a molecule that is composed of hundred or more amino acids. In contrast the term "peptide" is defined as a molecule that is composed of less than hundred amino acids. The term peptide therefore includes dipeptides, tripeptides, tetrapeptides pentapeptides, hexapeptides, heptapeptides, octapeptides, longer oligopeptides and amino acid chains with up to 99 amino acids. The amino acid chain can be in a linear or a branched form.

Amino acids in the context of the present invention are organic compounds that contain at least one amino and at least one carboxyl group which in the protein form an amide bond. The amino acids that are part of the protein can be taken from the 20 standard alpha-amino acids or non-standard amino acids, like selenocysteine, pyrrolysine, lanthionine, hydroxyproline, carboxygluatamate, 2-aminoisobutyric acid, dehydroalanine. Also other amino acids like beta-amino acids (f.e. beta alanine), gamma amino acids or even higher amino acids could be part of the protein.

For certain amino acids different enantiomeric or stereoisomeric form could be choosen, like e.g. for the alpha amino acids that exist as two enantiomers, the D form and the L-form.

The protein may be manufactured synthetically (by means of organic synthesis in a cell-free system that makes use of a ribosome system that may or may not be artificial) or it may be manufactured naturally by cells that may or may not be genetically manipulated, or by a single cell organism (e.g. by a bacterium or a yeast cell) or a multicellular organism.

The protein could be modified in many ways. Typical modifications are post-translational modifications that include lipids, acetate groups, phosphate groups, or carbohydrates. The protein could also chemically modified with e.g. biotin groups.

The protein can be a multimer consisting of identical subunits (homomer) or different proteins (heteromer) which are hold together by covalent bonds or non-covalent interactions.

In a further embodiment the L-aspartate-D-alanine dipeptide is linked to at least one organic molecule other than an amino acid. Examples for suitable organic molecules include carboxylic acids, amines, amides, ketones, aldehydes, saturated or aromatic ring systems including heterocycles.

In one embodiment of the invention the compound A as part of the pharmaceutical composition represents an L-aspartate-D-alanine dipeptide of the following formula (I): 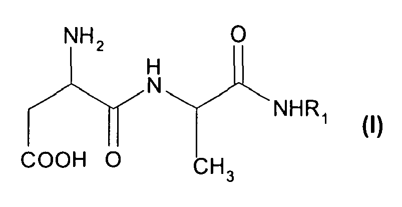 wherein R₁ is methyl, ethyl, n-propyl, isopropyl, n-butyl, isobutyl, secondary butyl, cyclohexyl, the carbon residue of the methyl esters of glycine, D-alanine or L-alanine or a branched member selected from the group consisting of fenchyl, diisopropylcarbinyl, d-methyl-tert-butylcarbinyl, d-ethyl-tert.-butylcarbinyl, di-tert.-butylcarbinyl, or 2-methylthio 2,4-dimethylpentan-3-yl.

In a further embodiment of the above listed L-aspartyl-D-alanine derivatives according to formula (I) are used as a salt, solvent or hydrate.

Mazur et al., J. Med. Chem., 16, 1284 (1973) has disclosed that lower alkyl esters of L-aspartyl-D-alanine and certain homologues thereof, especially L-aspartyl-D-alanine isopropyl ester, have sweetness potencies of up to 125 times sucrose.

Furthermore, Sukehiro et al., Seikatsu Kagaku, 11: 9-16 (1977); Chem. Abstr., 87: 168407h (1977) has disclosed certain amides of L-aspartyl-D-alanine of the formula (I): 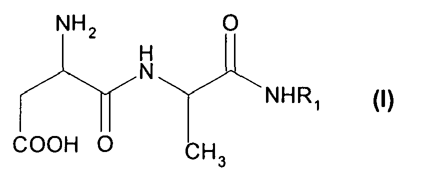 where R₁ is methyl, ethyl, n-propyl, isopropyl, n-butvl, isobutyl, secondary butyl, cyclohexyl or the carbon residue of the methyl esters of glycine, D-alanine or L-alanine. The most potent compounds were those wherein R₁ is one of the above butyl groups of cyclohexyl, having respectively, 100-125 and 100 times the sweetness of sucrose.

The European patent EP 0 034 876 B1 relates to branched amides of L-aspartyl-D-alanine dipeptides wherein R₁ is a branched member selected from the group consisting of fenchyl, diisopropylcarbinyl, d-methyl-t-butylcarbinyl, d-ethyl-t-butylcarbinyl, di-t-butylcarbinyl, or 2-methylthio2,4-dimethylpentan-3-yl.

Sweet tasting dipeptides containing L-Asp-D-ala are also described by Zhou et al. (J. Agric. Food. Chem. 1991, 39: 782-785).

The respective description of Mazur et al., Sukehiro et al., EP 0 034 876 B1 and Zhou et al. is hereby incorporated herein by reference and forms part of the disclosure.

In a further embodiment of the invention the pharmaceutical composition comprises a component A which is selected from the group consisting of α-L-aspartyl-D-alanine (R)-α-methylthiomethylbenzylamide, L-aspartyl-D-alanine isopropyl ester, L-aspartyl-D-alanyl-2,2,5,5-tetramethylcyclopentanyl ester [L-Asp-D-Ala-OTMCP] and N-alpha-Benzyloxycarbonyl-L-Asp-D-Ala-OBenzoyl.

In a further embodiment of the above listed L-aspartyl-D-alanine derivatives are used as a salt, solvent or hydrate.

D-Asp-D-Ala derived sweeteners as represented by the dipeptide α-L-aspartyl-D-alanine (R)-α-methylthiomethylbenzylamide are disclosed by EP 0 818 463 A1, the dipeptide L-aspartyl-D-alanyl-2,2,5,5-tetramethylcyclopentanyl ester [L-Asp-D-Ala-OTMCP] is disclosed by Benedetti et al. 1995 (J. Pept. Sci. 1(6): 349-359) and the dipeptide Z-L-Asp-D-AlaOBenzoyl is disclosed by Baek et al. 2004 (Appl. Environ. Microbiol. 70(3): 1570-1575). The respective description of EP 0 818 463 A1, Benedetti et al. and Baek et al. is hereby incorporated herein by reference and forms part of the disclosure.

In a preferred embodiment of the invention the pharmaceutical composition comprises alitame or a salt ,solvate or hydrate thereof as component A.

Among the above described second-generation peptide sweeteners alitame represent the most relevant compound. Alitame ((3S)-3-amino-4-[ [(1R)-1-methyl-2-oxo-2-[(2,2,4,4-tetramethyl-3-thietanyl)amino] ethyl]amino]-4-oxobutanoic acid) is an artificial sweetener and a an aspartic acid-containing dipeptide. Alitame exhibits the following structural formula:

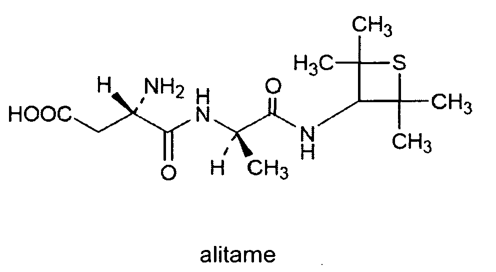

Alitame has several distinct advantages over aspartame. It is about 2000 times sweeter than sucrose, about 10 times sweeter than aspartame, and has no aftertaste. Unlike the bitter L-phenylalanine the amino acid D-alanine is sweet in itself so that also the byproducts and degradation products of alitame exhibit a sweet taste. The half-life of alitame under hot or acidic conditions is about twice as long as aspartame's. Unlike aspartame, alitame does not contain phenylalanine, and can therefore be used by people with phenylketonuria. Alitame is currently marketed in several countries under the brand name Aclame.

In a preferred embodiment the pharmaceutical composition according to the invention comprises one of the following combinations:
● a salt of 4-phenylbutyrate with a positively charged ion of component A; including a respective polymorph, hydrate or solvate of the salt;
● a salt of 4-phenylbutyrate with a positively charged ion of component A;
● a polymorph of a salt of 4-phenylbutyrate with a positively charged ion of component A;
● a hydrate of a salt of 4-phenylbutyrate with a positively charged ion of an component A;
● a solvate of a salt of 4-phenylbutyrate with a positively charged ion of component A;

"Polymorphs" in general are described in "Polymorphism in Pharmaceutical Solids", Harry G. Brittain (ed.) Drugs and the Pharmaceutical Sciences 192 (1999).

In a most preferred embodiment the pharmaceutical composition according to the invention comprises one of the following combinations:
● a salt of 4-phenylbutyrate with a positively charged ion of alitame; including a respective polymorph, hydrate or solvate of the salt;
● a salt of 4-phenylbutyrate with a positively charged ion of alitame;
● a polymorph of a salt of 4-phenylbutyrate with a positively charged ion of alitame;
● a hydrate of a salt of 4-phenylbutyrate with a positively charged ion of alitame;
● a solvate of a salt of 4-phenylbutyrate with a positively charged ion of alitame;

In a further preferred embodiment the pharmaceutical composition according to the invention comprises one of the following combinations:
● a co-crystal of 4-phenylbutyric acid and component A as co-crystal former including a respective polymorph, hydrate or solvate of the co-crystal;
● a co-crystal of 4-phenylbutyric acid and component A as co-crystal former as co-crystal former;
● a polymorph of a co-crystal of 4-phenylbutyric acid and component A as co-crystal former;
● a hydrate of a co-crystal of 4-phenylbutyric acid and component A as co-crystal former; or
● a solvate of a co-crystal of phenylbutyric acid and component A as co-crystal former.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction, which is neither ionic nor covalent and includes for example: hydrogen bonds, van der Waals forces, and S-S-interactions. Solvates or salts of phenyl butyric acid and an organic compound that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In the scientific literature there is currently some discussion on the proper use of the word co-crystal (see for example Desiraju, CrystEngComm, 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

A "component A as cocrystal former" as used herein is defined as any compound A, preferably a peptide or protein, more preferably a dipeptide or its salt which is able to form a co-crystal with 4-phenylbutyric acid or its salts.

In a preferred embodiment the pharmaceutical composition comprises one of the above listed co-crystals, wherein the difference in pKA between 4-phenylbutyric acid and component A does not exceed 2.7.

In the most preferred embodiment the pharmaceutical composition according to the invention comprises one of the following combinations:
● a co-crystal of 4-phenylbutyric acid and alitame as co-crystal former including a respective polymorph, hydrate or solvate of the co-crystal;
● a co-crystal of 4-phenylbutyric acid and alitame as co-crystal former as co-crystal former;
● a polymorph of a co-crystal of 4-phenylbutyric acid and alitame as co-crystal former;
● a hydrate of a co-crystal of 4-phenylbutyric acid and alitame as co-crystal former; or
● a solvate of a co-crystal of phenylbutyric acid and alitame as co-crystal former.

In a further embodiment the pharmaceutical composition according to the invention comprises one of the following combinations:
● a co-crystal of a salt of 4-phenylbutyrate with a positively charged ion of component A and at least one organic molecule as co-crystal former including a respective polymorph, hydrate or solvate of the co-crystal;
● a co-crystal of a salt of 4-phenylbutyrate with a positively charged ion of component A and at least one organic molecule as co-crystal former;
● a polymorph of a co-crystal of a salt of 4-phenylbutyrate with a positively charged ion of component A and at least one organic molecule as co-crystal former;
● a hydrate a co-crystal of a salt of 4-phenylbutyrate with a positively charged ion of component A and at least one organic molecule as co-crystal former; or
● a solvate a co-crystal of a salt of 4-phenylbutyrate with a positively charged ion of component A and at least one organic molecule as co-crystal former.

In a preferred embodiment the pharmaceutical composition comprises one of the above listed co-crystals, wherein the difference in pKA between 4-phenylbutyric acid and the organic molecule representing the co-crystal former does not exceed 2.7.

In a further embodiment the pharmaceutical composition according to the invention comprises one of the following combinations:
● a co-crystal of a salt of 4-phenylbutyrate with alitame and at least one organic molecule as co-crystal former including a respective polymorph, hydrate or solvate of the co-crystal;
● a co-crystal of a salt of 4-phenylbutyrate with a positively charged ion of alitame and at least one organic molecule as co-crystal former;
● a polymorph of a co-crystal of a salt of 4-phenylbutyrate with alitame and at least one organic molecule as co-crystal former;
● a hydrate a co-crystal of a salt of 4-phenylbutyrate with alitame and at least one organic molecule as co-crystal former; or
● a solvate a co-crystal of a salt of 4-phenylbutyrate with alitame and at least one organic molecule as co-crystal former.

In a further preferred embodiment of the joined mixture according to the invention the organic molecule is selected from
● ammonia and amines,
● amino acids,
● carboxylic acids, or
● heterocylic compounds

Preferably, in a further preferred embodiment of the joined mixture according to the invention, the organic molecule is selected from
● ammonia;
● amines, like 2-(diethylamino)-ethanol, benethamine, benzathine, benzylamine, betaine (trimethylglycine), choline, deanol, diethanolamine, diethylamine (2,2'-iminobis(ethanol)), ethanolamine (2-aminoethanol), ethylenediamine, glucamines, like N-methyl-glucamine, hydrabramine, tromethamine, triethanolamine (2,2',2"-nitrolitris(ethanol)):
● carbocylic acids, like benzoic acid, citric acid, gentisic acid, glycolic acid, lactic acid, naphtoic acids, succinic acid, tartaric acid, or vanillic acid;
● D-aminoacids like D-alanine:
● L-amino acids like L-lysine or L-arginine; or
● heterocyclic compounds, like imidazoles, like 1 *H*-Imidazole; morpholines, like 4-(2-hydroxyethyl)-morpholine; piperazine; pyrrolidines, like 1-(2-hydroxyethyl)-pyrrolidine.

In a further preferred embodiment of the joined mixture according to the invention the organic molecule is selected from
● 2-(diethylamino)-ethanol,
● ammonia,
● benethamine,
● benzathine,
● benzoic acid,
● benzylamine,
● betaine (trimethylglycine),
● choline,
● citric acid,
● D-alanine,
● deanol,
● diethanolamine,
● diethylamine (2,2'-iminobis(ethanol)),
● ethanolamine (2-aminoethanol),
● ethylenediamine,
● gentisic acid,
● glucamines, like N-methyl-glucamine,
● glycolic acid,
● hydrabramine,
● imidazoles, like 1 *H*-Imidazole,
● lactic acid,
● L-amino acids like L-lysine or L-arginine,
● morpholines, like 4-(2-hydroxyethyl)-morpholine,
● naphtoic acids,
● piperazine,
● pyrrolidines, like 1-(2-hydroxyethyl)-pyrrolidine,
● succinic acid,
● tartaric acid,
● tromethamine,
● triethanolamine (2,2',2"-nitrolitris(ethanol)), or
● vanillic acid;
preferably is selected from
● 1-(2-hydroxyethyl)-pyrrolidine,
● 2-(diethylamino)-ethanol,
● 4-(2-hydroxyethyl)-morpholine,
● benethamine,
● benzathine,
● benzoic acid,
● benzylamine,
● betaine (trimethylglycine),
● choline,
● citric acid,
● deanol,
● diethylamine,
● ethanolamine,
● gentisic acid,
● glycolic acid,
● hydrabramine,
● lactic acid,
● L-arginine,
● L-lysine,
● naphtoic acids,
● N-methyl-glucamine,
● piperazine
● succinic acid,
● tartaric acid,
● tromethamine, or
● vanillic acid;
more preferably is selected from
● 1-(2-hydroxyethyl)-pyrrolidine, or
● 2-(Diethylamino)-ethanol,
● 4-(2-hydroxyethyl)-morpholine,
● benethamine,
● benzathine,
● benzoic acid,
● benzylamine,
● betaine (trimethylglycine),
● choline,
● citric acid,
● D-alanine,
● deanol,
● diethylamine,
● gentisic acid,
● glycolic acid,
● hydrabramine,
● lactic acid,
● L-arginine,
● L-lysine,
● naphtoic acids,
● *N*-methyl-glucamine,
● succinic acid,
● tartaric acid,
● tromethamine, or
● vanillic acid;
most preferably is selected from
● 1-(2-hydroxyethyl)-pyrrolidine, or
● 2-(diethylamino)-ethanol,
● 4-(2-hydroxyethyl)-morpholine,
● benethamine,
● benzathine,
● benzoic acid,
● benzylamine,
● citric acid,
● deanol,
● diethylamine,
● gentisic acid,
● glycolic acid,
● hydrabramine,
● lactic acid,
● naphtoic acids,
● *N*-methyl-glucamine,
● succinic acid,
● tartaric acid,
● tromethamine, or
● vanillic acid;
● or - optionally - D-alanine.

In another embodiment of the invention the pharmaceutical co-crystal exhibits a molecular ratio between 4-phenyl-butyric acid and compound A is between 4:1 and 1:4, preferably between 2:1 and 1:2, and more preferably is 1:1.

In an alternative preferred embodiment of the pharmaceutical composition according to the invention the pharmaceutical composition is present in crystalline form. In a preferred embodiment this pharmaceutical composition is a polymorph of this crystalline form.

In an alternative preferred embodiment of the invention the pharmaceutical composition is in amorphous form.

In another alternative preferred embodiment of the invention the pharmaceutical composition is in liquid form.

A further aspect of the invention refers to a pharmaceutical composition comprising compound A and compound B and optionally at least one physiologically acceptable excipient.

A further aspect of the invention refers to the pharmaceutical composition according to the invention for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy; or also in the treatment of Alzheimer's disease.

A further aspect of the invention refers to the pharmaceutical composition according to the invention (or also the use of the pharmaceutical composition according to the invention in the production of a medicament) for use in the treatment of a genetic disorder or epigenetic disorder which are selected from or manifests itself in symptoms or diseases selected from urea cycle disorders, thalassemia, cystic fibrosis, rheumatoid arthritis, Siogren' s syndrome, uveitis, polymyositis, and dermatomyositis, arteriosclerosis, amyotrophic lateral sclerosis, asociality, affective disorders, systemic lupus erythematosus, immune response, varicosis, vaginitis, including chronic recurrent yeast vaginitis, depression or Sudden Infant Death Syndrome; especially in the treatment of cardiovascular disease like those being selected from arteriosclerosis, atherosclerosis, coronary heart disease, coronary artery disease, ischemic heart disease, angina, heart attack, heart failure, stroke and hypertension; or cystic fibrosis; or immune disease or autoimmune disease, like those being selected from rheumatoid arthritis, psoriatic arthritis, siogren's syndrome, uveitis, polymyositis, dermatomyositis, systemic lupus erythematosus, Crohn's disease, Graves' disease, Guillain-Barre syndrome, Hashimoto's thyroiditis, idiopathic thrombocytopenic purpura, psoriasis, ulcerative colitis, vasculitis and ankylosing spondylitis; or neurological or neurodegenerative diseases like those being selected from Rubinstein-Taybi syndrome, Rett syndrome, Friedreich's ataxia, Huntigton's disease, Parkinson's disease, amyotrophic lateral sclerosis and depression, or also in the treatment of Alzheimer's disease.

In a further aspect, the invention also refers to a method of treating a patient suffering from any of the above mentioned diseases by applying a suitable/physiologically effective amount of the pharmaceutical composition according to the invention.

The pharmaceutical composition according to the invention can be of any form suitable for the application to humans and/or animals, preferably humans including infants, children and adults and can be produced by standard procedures known to those skilled in the art. The pharmaceutical composition can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The pharmaceutical composition of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Pharmaceutical formulation according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical formulation according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the pharmaceutical compositions according to the present invention may contain 1-60 % by weight of one or more of the salts or their crystalline form as defined herein and 40-99 % by weight of one or more auxiliary substances (additives/excipients).

The pharmaceutical composition of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance (joined mixture according to the invention) to be administered during one or several intakes per day.

In a very preferred embodiment the pharmaceutical formulation of the invention is an oral pharmaceutical composition. Very suitable oral pharmaceutical compositions comprising 4-phenyl-butyric acid or a physiologically acceptable salt thereof are disclosed in EP1427396 (WO2003/22253), the content of which forming herewith part of the description/disclosure of the current invention. Accordingly, it is very preferred if such an oral pharmaceutical composition comprising a joint mixture according to the invention is formulated in an analoguous way to the formulation presented in EP1427396 (W02003/22253). It is thus further very preferred if an oral medicament according to the invention especially a pharmaceutical composition comprises 100 to 2000 mg of the combination of component A and component B according to the invention, preferably 200 to 1000 mg, most preferably approximately 500 mg or 250 mg of the combination of component A and component B according to the invention.

In another alternative embodiment the pharmaceutical composition is formulated as a tablet wherein each tablet contains 100 to 2000 mg of the combination of component A and component B according to the invention, preferably 500 to 1000 mg of the combination of component A and component B according to the invention. Preferably, the granulate further comprises microcrystalline cellulose, magnesium stearate and/or colloidal anhydrous silica.

In another alternative embodiment the pharmaceutical composition is formulated as a granulate wherein each gram of granulate contains 100 to 1000 mg of the combination of component A and component B according to the invention, preferably 500 to 900 mg of the combination of component A and component B according to the invention. Preferably, the granulate further comprises microcrystalline cellulose, calcium stearate and/or colloidal anhydrous silica.

Another aspect of the invention refers to a process for the production of the pharmaceutical composition according to the invention comprising either a salt or a co-crystal of 4-phenyl-butyric acid with the following steps:
a) 4-phenyl-butyric acid is dissolved in organic solvent 1 to form solution 1;
b) the compound A is dissolved in organic solvent 2 to form solution 2;
c) solution 1 and solution 2 are mixed;
d) the organic solvents are removed from the mixture from step c) and a residue is obtained;

In an embodiment of the process according to the invention the following steps are added after step d):
e) the residue from step d) is dissolved in organic solvent 3;
f) the resulting mixture is stirred while the temperature is raised above room temperature followed by lowering of the temperature;
g) in a last final step the organic solvent is removed from the mixture from step f) and the joined mixture obtained.

In an embodiment of the process according to the invention at least one pharmaceutically acceptable excipient is added after step d) or step g), respectively.

In a preferred embodiment of the process according to the invention:
● compound A is alitame
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step f) is raised to 25 to 35°C, preferably to approximately 30°C; and/or
● where applicable - the temperature in step f) is lowered to below 25°C, preferably to approximately 20°C; and/or
● in the first and/or the last final step the organic solvent is removed under gas flow.

"THF" is Tetrahydrofuran; "EtOH" is ethanol; "MeCN" is cyanomethan/acetonitril; "TBME" is tert.-butyl-methyl ether; and "EtOAc" is ethyl acetate.

In another embodiment and aspect of the invention, the pharmaceutical composition comprises a salt of 4-phenyl-butyric acid with a salt-forming organic molecule. In one embodiment the salt of 4-phenyl-butyric acid with the salt-forming organic molecule is in amorphous or crystalline form, preferably is in crystalline form.

"Salt-forming organic molecule" as used herein is defined as any organic molecule, preferably an amine, amino-acid or heterocycle, preferably an amine, which is able to become ionized and with which phenyl butyric acid or its derivative is able to form a salt.

In an embodiment of the invention the salt of 4-phenyl-butyric acid is an organic salt according to general formula II: 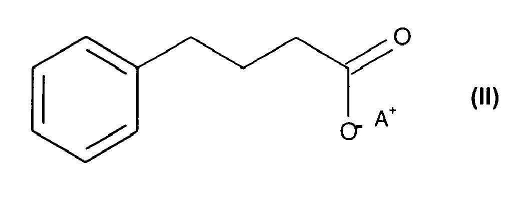 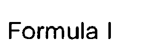 , wherein
A⁺ is selected from positively charged ions of a salt-forming organic molecule.

In a further embodiment this joint mixture according to the invention being a salt is a hydrate or solvate of a salt of 4-phenyl-butyric acid with a salt-forming organic molecule.

"Hydrate" is defined as a substance/joint mixture according to the invention containing water. Thus, it may be a crystalline salt with one or more water molecules in the crystalline structure or also amorphous containing water molecules.

"Solvate" is defined as a substance/joint mixture according to the invention containing a further solvent. One most prominent example of the solvents is an alcohol like ethanol or methanol thus leading to methanolates or ethanolates. Thus, it may be a crystalline salt with one or more alcohol molecules in the crystalline structure or also an amorphous form containing alcohol molecules.

In another embodiment the joint mixture according to the invention being a salt is a polymorph of a salt of 4-phenyl-butyric acid with a salt-forming organic molecule or of its hydrate or solvate.

In a very preferred embodiment and aspect of the invention in this salt of 4-phenyl-butyric acid with a salt-forming organic molecule according to the invention, the salt-forming organic molecule is selected from
● benethamine,
● benzathine,
● benzylamine,
● N-methyl-glucamine,
● 4-(2-hydroxyethyl)-morpholine, or
● 1-(2-hydroxyethyl)-pyrrolidine.

In one embodiment and aspect of the invention, in this salt of 4-phenyl-butyric acid with a salt-forming organic molecule according to the invention, the salt-forming organic molecule is choline. Preferably, in this salt of 4-phenyl-butyric acid with choline the molecular ratio between 4-phenyl-butyric acid and choline is between 4:1 and 1:4, more preferably is between 2:1 and 1:2 or is 1:1.

"Molecular ratio" according to this invention as a whole is defined as the ratio - if comparing molecule with molecule - of the amount of molecules of phenyl-butyric acid (or its ion in a salt) as one molecule on one hand and the amount of the other organic molecules in the joint mixture on the other hand. E.g. in the preceding paragraph it is the ratio of the amount of molecules of 4-phenyl-butyric acid (thus in its ionic form) on one hand compared to the amount of molecules of choline (thus in its ionic form) on the other hand.

Another aspect of the invention refers to a process for the production of a cocrystal using a salt of 4-phenyl-butyric acid with compound A and at least one organic molecule as co-crystal-former with the following steps:
a) the salt of 4-phenyl-butyric acid with compound A is dissolved in organic solvent 1 to form solution 1;
b) at least one organic molecule is dissolved in organic solvent 2 to form solution 2;
c) solution 1 and solution 2 are mixed;
d) the organic solvents are removed from the mixture from step c) and a residue is obtained.

Optionally, the following steps are added after step d):
e) the residue from step d) is dissolved in organic solvent 3;
f) the resulting mixture is stirred while the temperature is raised above room temperature followed by lowering of the temperature;
g) in a last final step the organic solvent is removed from the mixture from step f) and the co-crystal is obtained.

Preferably, in that process:
● compound A is alitame
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step f) is raised to 25 to 35°C, preferably to approximately 30°C; and/or
● where applicable - the temperature in step f) is lowered to below 25°C, preferably to approximately 20°C; and/or
● in the first and/or the last final step the organic solvent is removed under gas flow.

Most preferably, in that process the organic solvents 1 and 2 are the same and are MeCN.

In a very preferred embodiment and aspect of the invention, in this salt of 4-phenyl-butyric acid with compound A according to the invention, the compound A is alitame. Preferably, in this salt of 4-phenyl-butyric acid with alitame the molecular ratio between 4-phenyl-butyric acid and alitame is between 4:1 and 1:4, more preferably is between 3:1 and 1:1. Most preferred is a salt of 4-phenyl-butyric acid with alitame according to the invention, wherein the molecular ratio between 4-phenyl-butyric acid and alitame is 2:1. In another preferred embodiment the salt of 4-phenyl-butyric acid with alitame is hydrated, is a hydrate, e.g. mono-hydrate, di-hydrate etc. In another embodiment the salt of 4-phenyl-butyric acid with alitame is in amorphous or crystalline form, preferably is in crystalline form. In another preferred embodiment the salt of 4-phenyl-butyric acid with alitame is containing a solvent molecule thus being a "solvate" (see above). In another preferred embodiment the salt of 4-phenyl-butyric acid with alitame is a hydrate, e.g. mono-hydrate, di-hydrate etc., preferably is a hydrate. In another preferred embodiment the salt of 4-phenyl-butyric acid with alitame is existing in form of a polymorph of any of the salts, solvates, or hydrates.

In a very preferred embodiment and aspect of the invention in this co-crystal of 4-phenyl-butyric acid with a co-crystal-forming compound A according to the invention, the co-crystal-forming compound A is an L-aspartate-D-arginine dipeptide derivative, preferably a dipeptide acid selected from
● α-L-aspartyl-D-alanine (R)-α-methylthiomethylbenzylamide,
● L-aspartyl-D-alanine isopropyl ester,
● L-aspartyl-D-alanyl-2,2,5,5-tetramethylcyclopentanyl ester [L-Asp-D-Ala-OTMCP],
● and N-alpha-Benzyloxycarbonyl-L-Asp-D-Ala-OBenzoyl,

In a very preferred embodiment and aspect of the invention, in this co-crystal of 4-phenyl-butyric acid (or derivative or salt thereof) with a co-crystal-forming compound A according to the invention, the co-crystal-forming-forming organic molecule is alitame. In an embodiment the molecular ratio between 4-phenyl-butyric acid (or anion thereof) and the alitame is between 4:1 and 1:4. In another embodiment the co-crystal does contain at least one further molecule being selected from water (a hydrate), solvents like alcohols (methanol, ethanol) (a solvate or alcoholate) or a cation. In an embodiment, any of these co-crystals according to the invention exist in form of a polymorph.

Another aspect of the invention refers to a pharmaceutical formulation comprising a co-crystal according to the invention being either generally a co-crystal of 4-phenyl-butyric acid (or derivative or salt thereof) with a co-crystal-forming compound A or a co-crystal of 4-phenyl-butyric acid (or derivative or salt thereof) with a co-crystal-forming organic molecule being alitame, respectively and optionally at least one physiologically acceptable excipient.

The present invention is illustrated below with the help of the following examples. These illustrations are given solely by way of example and do not limit the invention.

### EXAMPLES:

### Example 1: Slow Release Tablets comprising 4-Phenylbutyrate-Sodium salt and Alitame

1,760.0 g of 4-phenylbutyrate-mono-benethamine salt, 1,139.0 Lactose, 704 g Alitame, 1026 g Hydroxyprpylmethylcellulose, and 240 g of microcrystalline cellulose is mixed. This weighing-in and mixing process is repeated twice resulting in a combined mixture of approx. 15 kg. This mixture is wetted with 3 times 2,000.0 g of aqua purificata (water purified by inversion osmosis) and dried. The mixture is forced through a sieve and dried again. 3 x 70.4 g of talcum and 179.2 g of sieved magnesium stearate is admixed and the mixture/granulate is pressed into tablets (cores) of about 0.710 g each, a length of about 18.8 mm and thickness of about 8 mm and a hardness in average of 120 - 240 N.

The mixing is carried out with a J. Engelsmann Rhönrad Mixer, the drying in a Glatt WSG 5 drying cabinet, the sieving with a Erweka sieving machine, and the tablet pressing with a tablet press PF 1. The cores are provided with a film coating by using a colloidal dispersion containing 899 g of Eudragit L 100, 450 g triethylcitrate, 86.0 g of 10% NH₃-Solution, 450 g talc suspended in 7,150 g aqua purificata. The suspension is sprayed at 100 - 250 mbar onto the cores at around 70°C. A Glatt coater is used. The film-coated tablets with an average weight around 752.7 mg are dried.

## Claims

1. A pharmaceutical composition comprising:
(a) component A: a substance comprising an L-aspartate-D-alanine dipeptide or a salt or a solvate thereof; and
(b) component B: 4-phenyl-butyric acid or a salt or solvate thereof.

2. A pharmaceutical composition according to claim 1, wherein component A exhibits a structure according to formula (I): 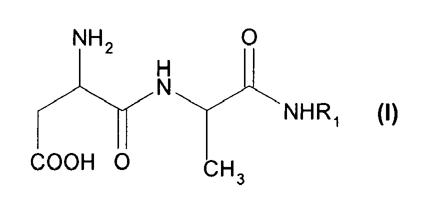 wherein R₁ is methyl, ethyl, n-propyl, isopropyl, n-butyl, isobutyl, secondary butyl, cyclohexyl, the carbon residue of the methyl esters of glycine, D-alanine or L-alanine or a branched member selected from the group consisting of fenchyl, diisopropylcarbinyl, d-methyl-tert-butylcarbinyl, d-ethyl-tert.-butylcarbinyl, di-tert.-butylcarbinyl, or 2-methylthio 2,4-dimethylpentan-3-yl; including a salt or solvate of said structure.

3. A pharmaceutical composition according to claim 1, wherein component A is selected from the group consisting of α-L-aspartyl-D-alanine (R)-α-methylthiomethylbenzylamide, L-aspartyl-D-alanine isopropyl ester, L-aspartyl-D-alanyl-2,2,5,5-tetramethylcyclopentanyl ester [L-Asp-D-Ala-OTMCP] and N-alpha-Benzyloxycarbonyl-L-Asp-D-Ala-OBenzoyl including a salt or solvate of the selected compound.

4. A pharmaceutical composition according to claim 1, wherein component A is alitame or a salt or solvate thereof.

5. A pharmaceutical composition according to any of the above claims, **characterized in that** the pharmaceutical comprises one of the following combinations:
a. a salt of 4-phenylbutyrate with a positively charged ion of component A including a respective polymorph, hydrate or solvate of the salt;
b. a salt of 4-phenylbutyrate with a positively charged ion of component A;
c. a polymorph of a salt of 4-phenylbutyrate with a positively charged ion of component A;
d. a hydrate of a salt of 4-phenylbutyrate with a positively charged ion of component A;
e. a solvate of a salt of 4-phenylbutyrate with a positively charged ion of component A;

6. A pharmaceutical composition according to any of the claims 1 to 4, **characterized in that** the pharmaceutical comprises a co-crystal of one of the following combinations:
a. a co-crystal of 4-phenylbutyric acid and component A as co-crystal former including a respective polymorph, hydrate or solvate of the co-crystal;
b. a co-crystal of 4-phenylbutyric acid and component A as co-crystal former;
c. a polymorph of a co-crystal of 4-phenylbutyric acid and component A as co-crystal former;
d. a hydrate of a co-crystal of 4-phenylbutyric acid and component A as co-crystal former; or
e. a solvate of a co-crystal of 4-phenylbutyric acid and component A as co-crystal former.

7. A pharmaceutical composition according to claim 6, wherein the difference in pKA between 4-phenylbutyric acid and component A does not exceed 2.7.

8. A pharmaceutical composition according to any of the claims 1 to 4, **characterized in that** the pharmaceutical comprises a co-crystal of one of the following combinations:
a. a co-crystal of a salt of 4-phenylbutyrate with a positively charged ion of component A and at least one organic molecule as co-crystal former including a respective polymorph, hydrate or solvate of the co-crystal;
b. a co-crystal of a salt of 4-phenylbutyrate with a positively charged ion of component A and at least one organic molecule as co-crystal former;
c. a polymorph of a co-crystal of a salt of 4-phenylbutyrate with a positively charged ion of component A and at least one organic molecule as co-crystal former;
d. a hydrate a co-crystal of a salt of 4-phenylbutyrate with a positively charged ion of component A and at least one organic molecule as co-crystal former; or
e. a solvate a co-crystal of a salt of 4-phenylbutyrate with a positively charged ion of component A and at least one organic molecule as co-crystal former.

9. A pharmaceutical composition according to claim 8, wherein the difference in pKA between 4-phenylbutyric acid and the organic molecule representing the co-crystal former does not exceed 2.7.

10. A pharmaceutical composition according to claim 6 or 8, wherein the compound A is alitame.

11. A pharmaceutical co-crystal according to any of claims 1 to 10, wherein the molecular ratio between 4-phenyl-butyric acid and compound A is between 4:1 and 1:4, preferably between 2:1 and 1:2, and more preferably is 1:1.

12. A pharmaceutical composition according to any of claims 1 to 11 wherein the pharmaceutical composition is in crystalline form, in amorphous form or in liquid form.

13. A pharmaceutical composition according to any of claims 1 to 12 comprising at least one physiologically excipient.

14. A pharmaceutical composition according to any of claims 1 to 13 for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy or in the treatment of Alzheimer's disease.

15. Process for the production of a pharmaceutical composition according to any of claims 6 and 8 with the following steps:
a) the co-crystal former is dissolved in organic solvent 1 to form solution 1;
b) the 4-phenylbutyric acid or its salt is dissolved in organic solvent 2 to form solution 2;
c) solution 1 and solution 2 are mixed;
d) the organic solvents are removed from the mixture from step c) and a residue is obtained; and
e) optionally at least one pharmaceutically acceptable excipient is added.
